# EUROPEAN PATENT SPECIFICATION

(11) **EP 3 380 107 B1**
(45) Date of publication and mention of the grant of the patent: **15.03.2023**
(21) Application number: 16869267.1
(22) Date of filing: 23.11.2016
(51) Int. Cl.: A61K 35/14, A61P 35/00, A61K 31/337, A61K 31/445, A61K 47/64

(54) **COMPOSITIONS AND METHODS OF USE OF NANO ANTI-RADICAL THERAPEUTICS TO INHIBIT CANCER**
ZUSAMMENSETZUNGEN UND VERFAHREN ZUR VERWENDUNG VON NANO-ANTIRADIKALEN THERAPEUTIKA ZUR HEMMUNG VON KREBS
COMPOSITIONS ET PROCÉDÉS D'UTILISATION D'AGENTS THÉRAPEUTIQUES DE TYPE NANOPARTICULES ANTIRADICALAIRES POUR INHIBER LE CANCER

(30) Priority: 24.11.2015 US 201562259485 P
(43) Date of publication of application: 03.10.2018
(73) Proprietor: Antiradical Therapeutics LLC, Sioux Falls, SD 57107-1363 (US)
(72) Inventor: HSIA, Jen-Chang, Laguna Woods, CA 92637 (US)
(74) Representative: Murgitroyd & Company
(86) International application number: PCT/US2016/063594
(87) International publication number: WO 2017/091722

(56) References cited:
- US-A- 5 840 701
- US-A1- 2002 013 263
- US-A1- 2002 013 263
- US-A1- 2004 024 025
- US-A1- 2015 094 267
- SHANTA M. MESSERLI ET AL: "Use of Antimetastatic SOD3-Mimetic Albumin as a Primer in Triple Negative Breast Cancer", JOURNAL OF ONCOLOGY, vol. 2019, 28 February 2019 (2019-02-28), pages 1-11, XP055602488, US ISSN: 1687-8450, DOI: 10.1155/2019/3253696
- YU et al.: "A pH dependent thermo-sensitive copolymer drug carrier incorporating 4-amino- 2,2,6,6-tetramethylpiperidin-1-oxyl (4-NH2-TEMPO) residues for electron spin resonance (ESR) labeling", Journal of Colloid and Interface Science, vol. 362, 7 July 2011 (2011-07-07), pages 584-593, XP028261837,
- SOULE et al.: "Therapeutic and Clinical Applications of Nitroxide Compounds", Antioxidant & Redox Signaling, vol. 9, no. 10, 10 November 2007 (2007-11-10), pages 1731-1743, XP055390502,
- SIROVICH et al.: "Activity of Ruboxyl, a Nitroxyl Derivative of Daunorubicin, on Experimental Models of Colorectal Cancer Metastases", Tumor Biology, vol. 20, no. 5, 23 July 1999 (1999-07-23), pages 270-276, XP009510786, DOI: http://www.karger.com/doi/10.1159/00003007 4
- DU et al.: "Regulation of Pancreatic Cancer Growth by Superoxide", Molecular Carcinogenesis, vol. 52, 5 March 2012 (2012-03-05), pages 555-567, XP055390615,
- Keith Block: "Immune Surveillance: Mounting the immune barricades" In: Keith Block: "Life Over Cancer: The Block Center Program for Integrative Cancer Treatment", 21 April 2009 (2009-04-21), XP009511222, ISBN: 9780553801149 pages 346-367, * ; page349 *
- YAMATO et al.: "Tempol intake improves inflammatory status in aged mice", Journal of Clinical Biochemistry and Nutrition, vol. 55, 24 May 2014 (2014-05-24), pages 11-14, XP003032460,
- NORRELL et al.: "Leukoencephalopathy following the administration of methotrexate into the cerebrospinal fluid in the treatment of primary brain tumors", Cancer, vol. 33, April 1974 (1974-04), pages 923-932, XP055390705,
- GARIBOLDIA et al.: "Study of in vitro and in vivo effects of the piperidine nitroxide Tempol-a potential new therapeutic agent for gliomas", European Journal of Cancer, vol. 39, 23 January 2003 (2003-01-23), pages 829-837, XP004414315,
- HASHIDA et al.: "Characterization of a Lipophilic Prodrug of 5-Fluorouracil with a Cholesterol Promoiety and lts Application to Liposomes", Chemical and Pharmaceutical Bulletin, vol. 36, 31 March 2008 (2008-03-31), pages 3186-3189, XP002063290,

## Description

### CROSS-REFERENCE TO RELATED APPLICATIONS

This application claims the benefit of provisional patent application numbered US 62/259,485.

### FIELD OF THE INVENTION

This invention relates to compositions and methods of use of novel macromolecular nitroxide which reduces morbidity or mortality in cancer through the inhibition of carcinogenesis, including metastasis.

### BACKGROUND - PRIOR ART

A breakthrough therapy for cancer has to be multifunctional in order to improve the survival of cancer patients. Ideally, these functions should reside in a single cancer treatment drug which works through the inhibition of cancer cell proliferation, progression and metastasis associated inhibition of reactive oxygen species, enhancement of blood flow and therapeutic index of standard cancer therapies.

We describe here the discovery of such a class of macromolecular nitroxide with serum albumin as the macromolecular carrier of the nitroxide for the reduction of morbidity and mortality in cancer patients.

In prior art, the treatment of metastatic cancer aims to slow the growth or spread of the cancer. Treatment depends on the type of cancer, where it started, the size and location of the metastasis, and other factors. Typically, metastatic cancer requires systemic therapy, or medications given by mouth or injected into the bloodstream to reach cancer cells throughout the body, such as chemotherapy or hormone therapy. Other treatments may include biological therapy, radiation therapy, immunotherapy, surgery, or a combination of these. Thus, development of a comprehensive treatment for carcinogenesis to improve the cure of cancer is needed. There is also a basic paradigm in the prior art that is invalidated by the present invention, namely the paradigm that therapeutic agents must be administered intravascularly or retained only in the bloodstream (intravascular space) in order to exert ant-cancer activity.

Prior art from this inventor has focused on the utility and therapeutic application of macromolecular nitroxide located in the intravascular space (US 8,273,857 ;US 62/259,485 ;US 14/472188 and PCT/US 14/53632.

To the best of the inventor's knowledge, prior art disclosure of use of small molecule weight nitroxide and macromolecular nitroxide, including that of this inventor's own, to counter the free radical based ROS activities have not advanced to the stage of simultaneously addressing all steps in the carcinogenesis process.

Hypoxia and a lack of blood flow into the hypoxic core of tumors is the root cause of cancer metastasis and tumor propagation. Lack of blood flow also causes resistance to drug therapy by blocking drug access. Existing cancer therapies do not have anti-hypoxia activity, lack catalytic vascular superoxide removal activity and do not increase blood flow into the core of tumors. One example of an existing therapy is nab-paclitaxel (also known as ABI-007; nanoparticle albumin-bound paclitaxel; trade name Abraxane^{®}). Although nab-paclitaxel is a carrier of the anti-microtubule drug paclitaxel, the bound paclitaxel has lowered or no bioavailability, and little or no drug efficacy, inside the tumor core.

There are numerous patents addressing the compositions and methods for the treatment of cancer carcinogenesis. However, to the best of knowledge of the inventor, there is no prior art covering the therapeutic efficacy of the present invention for the use of macromolecular nitroxide of the appropriate molecular size as a comprehensive treatment of carcinogenesis and metastasis. Furthermore, no prior art discovered that regulating the intracellular, extravascular and intravascular free radical based ROS activities is the unified therapeutic mechanism in the treatment of carcinogenesis and metastasis as claimed in this invention. In summary, against this background, the paradigm shift described in this invention on cancer therapy has been demonstrated with a single therapeutic agent i.e. VACNO.

Patent specification US 2015/094267 A1 describes succinimide-activated nitroxyl compounds and methods for the synthesis of such compounds, and also the use of succinimide-activated nitroxyl compounds to prepare nitroxylated proteins.

Patent specifications US 2002/013263 A1 and US 5840701 A describe compositions and processes to alleviate free radical toxicity based on the use of nitroxides in association with physiologically compatible macromolecules.

### SUMMARY

It will be appreciated that the scope of the invention is in accordance with the appended claims. Accordingly, there is provided a nitroxylated macromolecule for use in the treatment of carcinogenesis and metastasis in the treatment of cancer in cancer patients, as set out in the appended set of claims.

This invention relates to our discovery that in pre-clinical in vitro and in vivo models studies that the extracellular macromolecular nitroxide described herein strategically localizes throughout all the extravascular and intravascular volume along the path of cancer cell development to block metastasis. Said macromolecular nitroxide targets the reduction of reactive oxygen species (ROS) in the cancer metastasis process, thus creating a new cancer therapy called Nano-antiradicaltherapy (NanoART), which specifically target the prevention and treatment of metastasis to compliment the current cancer therapies. Novel compositions of macromolecular nitroxide and their methods of use are disclosed. In the present invention the nitroxylated albumin localizes within all body fluids following intraperitoneal injection. This finding disproves the paradigm of prior art cancer therapies that anti-cancer therapies must be administered intravascularly or must be retained in the bloodstream or intravascular space.. We found the treatment of cancer carcinogenesis using the formulation of our nitroxylated albumin leads to the inhibition of metastasis of cancer and formation of secondary tumors at other locations in the body.

We present results to show that in preclinical mouse model of triple negative breast cancer (TNBC) model, delivery of the nitroxylated albumin to all body compartments can be accomplished through intra peritoneal (IP) injection to achieve the desired therapeutic effect specifically targeting metastasis of TNBC cells from the flank tumor to the lung. Such route of cancer drug administration has to be considered to be a breakthrough therapy. Especially, this drug which was originally designed to treat stroke and sickle cell disease (SCD) has completed FDA IND safety studies and is essentially free of toxic side effects as compared to the standard cancer therapeutic treatment. This lack of toxicity would contribute both to the quality and longevity of life of cancer patients.

This inventor is gratified to make a contribution to cancer therapy via such a breakthrough therapeutic mechanism of macromolecular nitroxide which was originally developed as superoxide dismutase (SOD) mimetic for the treatment and restoration of blood flow without oxidative stress in diseases suffering from hypoxia in the vascular compartments. This therapeutic approach of nitroxylated albumin and hemoglobin was administered intravenously in prior art to create a superoxide free vascular compartment to facilitate blood flow and restore oxygen delivery without oxidative stress. In contrast, we have used IP administration in this present invention to demonstrate therapeutic efficacy in a TNBC mouse model to demonstrate inhibition of metastasis, which is a truly remarkable and novel discovery.

The present invention advances macromolecular nitroxides to expand their utility in both intravascular and extravascular spaces to intercept the metastasis and carcinogenesis of cancer, for the improvement of cure of cancer.

This invention is to demonstrate that the anti-carcinogenesis efficacy and mechanism of action of nano-antiradicaltherapy (NanoART) agents works in both the intravascular and extravascular space. An example is a multiple catalytic free radical nitroxides carried on a pol-ynitroxylated albumin, i.e. PNA aka vascular albumin with caged nitric oxide (VACNO), which is shown to work in the inhibition of cancer carcinogenesis in this invention. We present representative data in this invention to show that VACNO as a single molecule, in a dose dependent manner, is capable of inhibition of proliferation, progression and metastasis of in vitro and in vivo TNBC models. This invention demonstrates the remarkable accomplishment that these therapeutic activities reside in a single therapeutic agent, VACNO. This invention is only made possible from the inventor's willingness to switch away from the focus of PNA and polynitroxylated hemoglobin (PNH) utility in the intravascular space to BOTH the intravascular and extravascular space. The inventor's insight that the common denominator of these cancer development processes resides in the role of free radical based reactive oxygen species (ROS) and is also a key to this invention. Up to date, the role of ROS in carcinogenesis has not been fully addressed by the current standard cancer therapies. The inventor believes that the particular deficit of the current cancer therapies is the lack of understanding of compartmentalization of the ROS reactions, from intracellular to extravascular verses intravascular to extravascular space, in carcinogenesis. Therefore, we have employed VACNO as an example of NanoART to exert comprehensive therapeutic effects to cover the full carcinogenesis process driven by ROS activities. We have also strategically placed VACNO in all extracellular fluid of the body by demonstrating its efficacy in the inhibition of metastasis following intraperitoneal (IP) administration of this drug.

This invention is developed against the background of the successful clinical use of trastuzumab aka HERCEPTIN^{®}, a humanized monoclonal antibody directed against human epidermal growth factor receptor 2 (HER2) and Abraxane^{®}. We describe an example of a macromolecular nitroxide, VACNO, which has a much more broader therapeutic activities than HERCEPTIN^{®}. HERCEPTIN^{®} is limited for use in the inhibition of proliferation of HER2 positive breast cancer patients. In addition, VACNO nanoparticle can be used as a carrier or in conjunction with paclitaxel with higher therapeutic efficacy than Abraxane^{®}. The albumin in nab-paclitaxel (trade name Abraxane^{®}) is only a carrier for paclitaxel and has no known efficacy of its own in reducing hypoxia and metastasis of cancer. Clinical protocols for the use of Abraxane^{®} also specify that Abraxane^{®} must be administered intravenously and retained within the bloodstream. VACNO complexed with paclitaxel improves on nab-paclitaxel by (1) removal of the hypoxia in tumors in advanced cancer to block metastasis (2) enhancement of blood flow into the hypoxia region of the cancer to improve the delivery, availability and efficacy of bound paclitaxel (3) wider body distribution. Nitroxide labeling of albumin in VACNO complexed with paclitaxel results in control of superoxide levels in the bloodstream, leading to increased blood flow into tumors. This provides both anti-cancer progression and anti-metastasis activities in a single drug.

### DESCRIPTION OF THE DRAWINGS

Fig 1. Colony forming assays in VACNO treated 4T1 cultures. Cells were plated at low density and then treated with the indicated concentration of VACNO or HSA as a control. Colonies were stained and counted (A) or measured for size (B).
Fig 2. Effects of VACNO on cellular ROS levels. 4T1 cells were treated with VACNO or HSA as indicated, stained with DCF and analyzed by flow cytometry. Mean fluorescence intensity is shown. Lower fluorescence indicates lower ROS levels.
Fig 3. Expression of cyclin D1 in VACNO treated 4T1 cells. Cultured 4T1 cells were treated with HSA or VACNO as indicated and then harvested for western blotting.
Fig 4. Measurement of blood flow in 4T1 tumors in mice. Tumor bearing animals were injected with microbubbles and then with HSA or VACNO and time of tumor perfusion was measured by ultrasound. Lower time is indicative of faster blood flow through tumor.
Fig 5. Effect of VACNO on lung metastasis in 4T1 breast cancer model. A) Tumor bearing mice were treated with or without VACNO and after 3 weeks lungs were harvested for counting lung metastatic nodules. B) Same as A except that mice were also treated with doxorubicin. Metastatic nodules were significantly reduced with a P value <0.01.

### DETAILED DESCRIPTION-BEST MODE

### Section 1: Composition

VACNO is prepared by covalent labelling of serum albumin as the macromolecule using chemically reactive nitroxides (not shown). The preferred embodiment is an albumin molecule derived with 40-60 nitroxides per albumin molecule. VACNO may also be subsequently formulated as a complex with a chemotherapeutic drug such as paclitaxel where VACNO also acts as a carrier for the hydrophobic drug paclitaxel to increase its bioavailability. VACNO formulated with bound paclitaxel is used as a solution or is lyophilized or is formed into 100-200 nanoparticles using a high pressure homogenizer. For lyophilized VACNO complexed with paclitaxel, after re-constitution with saline each dose will contain 900mg of albumin, 100 mg of paclitaxel, and 150 mg nitroxide in 20 ml. VACNO is administrated by intraperitonal injection as one example embodiment.. VACNO, or VACNO complexed with paclitaxel or other drug is administered by medical specialists into any body compartment. In one preferred embodiment VACNO is administered by intraperitoneal injection. In a second preferred embodiment VACNO is administered by intravenous injection.

Polynitroxyl labelling of albumin confers the anti-hypoxic activities of the invention and the increase in blood flow into the hypoxic core of cancer. Binding pacilitaxel to VACNO: the bound paclitaxel when delivered to tumor cells provides the anti-mitotic and anti-progression cancer drug activities of the drug. VACNO complexed with paclitaxel nanoPNA-pacilitaxel manufactured and used as 100-200 nanoparticles is to provide an alternative formulation that minimizes extravasation from the bloodstream of albumin monomer. This will increase nanoPNA and paclitaxel levels in the bloodstream.

In the polynitroxylation of albumin, the number of nitroxides on the albumin molecule is tested and verified to be between 40-60 nitroxides per molecule of albumin. This is tested by the electron paramagnetic resonance (EPR) method to optimize the drug formulation. Then the loading factor of bound paclitaxel needs to be tested and optimized by HPLC analysis. Finally the size of the nanoparticle obtained by high pressure homogenization needs to be tested and optimized.

The formulation will be used for anti-progression and anti-metastasis cancer therapy to reduce mortality. The invention achieves its desired result because of its anti-hypoxic activity, its ability to restore blood flow, and because of simultaneous delivery of the anticancer drug paclitaxel into the hypoxic region of the cancer because of enhancement of blood flow. As a result of increased blood flow, nanoPNA-paclitaxel mediated delivery of the drug paclitaxel or other anti-cancer drugs may show equal or greater anti-mitotic efficacy to that obtained using unmodified albumin or other drug carrier.

VACNO would be used clinically alone or in combination with other therapies, such as chemotherapy, immunotherapy, surgery or radiation therapy to increase the survival of cancer patients.

### Section II: Anti-Cancer Activities of VACNO and Methods of Use

We present results to show that the inhibition of the proliferation of TNBC breast cancer cell line 4T1 in vitro by VACNO is dose dependent and is in the micromolar range. This anti-proliferation activity is also dose dependently related to the reduction of intracellular ROS of the 4T1 cell line. This anti-proliferation activity is also associated with down regulation of cyclin D1, a marker of cancer proliferation. We have also demonstrated that VACNO is efficacious in inhibiting the proliferation of mEER and MLM3 cell lines of head neck squamous cell carcinomas (HNSCC) cancer (results not shown). These results together support the inventor's claim in this patent that VACNO has broad anti-proliferation activities against cancers with solid tumor. The mechanism of action of VACNO is through the reduction of their intracellular ROS of these cancer cells and track them down throughout the body fluid to inhibit metastasis. The obvious utility of this invention is that NanoART drugs like VACNO can be used as therapeutic agent for a broad range of cancer with solid tumor. Specifically, VACNO's efficacy can be tested in TNBC patients, which is beyond the scope of HERCEPTIN^{®} treatment of HER2 positive breast cancer patients.

Of particular additional utility in VACNO for breast cancer therapy, is to focus on the treatment of the 10%-20% of the TNBC breast cancer patients because they don't have estrogen and progesterone receptors and also don't overexpress the HER2 protein. Most breast cancers associated with the gene BRCA1 are triple-negative which make the patient enrollment for clinical trials easily selected for VACNO therapy.

The TNBC patients generally respond well to chemotherapy given after surgery. But the cancer tends to come back. So far, no targeted therapies have been developed to help prevent cancer returning in women with TNBC. Against this un-met therapeutic need, the inventor proposes that VACNO can be tested in TNBC patients with recurrent metastasis. The clinical trials will target the inhibition of metastasis by monitoring the VACNO anti-proliferation activity measured by reduction of cyclin D1 level as surrogate markers to measure and predict that the return of the breast cancer is being blocked.

However, this inventor like to point out that VACNO has a much broader application than HERCEPTIN^{®} for the treatment of breast cancer that are not HER2 positive. HERCEPTIN^{®} application is specific and limited to the anti-proliferation level. Based on the data presented in this invention, the VACNO therapeutic activities have been shown to extend beyond proliferation to cancer progression and metastasis to a broad range of cancers with solid tumor e.g. HNSCC. Therefore, we have thoughtfully proposed VACNO as an improvement of cure for cancer and carefully examined against the background of how a cancer cell proliferate, progress in the formation of a solid tumor and how it escapes from the original location of the tumor to metastasize to multiple new locations to form new tumors can all be treated comprehensively by VACNO.

In this invention we have proposed compositions and methods for improving the cure of cancer with VACNO as an example of NanoART as it is described in the FIELD OF INVENTION above. Furthermore, we have presented experimental data and described in this invention that a unified therapeutic mechanism for carcinogenesis. We have tested and proven in well established cancer cell lines in pre-clinical models the efficacies of VACNO against the treatment of the current standard therapies for cancer.

This invention assumes that efficacies of VACNO can be monitored at all steps of the carcinogenesis on surrogate markers like cyclin D1 and hypoxia inducible factor 1a (Hif1a) and the results can be correlated from both in vitro and in vivo studies in clinical relevant cancer therapies.

VACNO has the efficacy to create a free radical free vascular space to restore or improve the blood flow to the hypoxic core of the solid tumor either in the original sites or at new metastasized locations.

The novelty of VACNO therapy is that the proliferation, progression and metastasis of cancer therapy can all be treated with a single therapeutic agent. This innovation is only possible from the inventor's insight that the common denominator of these cancer development process is driven by a free radical based ROS. This therapeutic approach is not fully addressed by the current standard cancer therapies. Of particular deficit of the current cancer therapies is the lack of appreciation of the compartmentalization of the ROS reactions from intracellular to extracelluar verses intravascular verses extravascular spaces. Especially the lack of understanding that ROS is the driving force of the carcinogenesis. Therefore, we have employed a catalytic free radical nitroxide carried on an albumin to exert its extracellular therapeutic effects to cover the full cancer proliferation, progression and metastasis processes. VACNO is designed to modulate ROS activities of the cancer cells in all compartments of the body fluid. The fact that 2/5th of the albumin is in the intravascular fluid and the remaining 3/5th of albumin is distributed in the extravascular fluid indicate that the presence of the VACNO in all body fluid is responsible for the following three key aspects of this drug:
1. The ability of the VACNO to inhibit the cancerous cell proliferation via the reduction of intracellular ROS is the key innovative therapeutic approach by being able to distinguish the difference in the role of ROS in cancerous cell vs normal cell proliferation. This observation is supported by the demonstration of the VACNO activity in inhibiting the proliferation of cancer cell line in vitro is correlated with down regulation of cyclin D1 level. These in vitro data would reflect the inhibition of cell cycling which can be dose related to in vivo animal models by measuring the cyclin D1 as a molecular marker. Thus we have established the key foundation of the feasibility of this innovative approach in future clinical trials to ascertain how VACNO can inhibit the cancer cells from establishing a new tumor site.
2.The inhibition of lung metastasis of flank breast tumor in vivo is a remarkable observation that the VACNO, when given extravascularly is present along the passage of cancer cell to inhibit metastasis. What is even more remarkable is that the metastasis of breast cancer is enhanced by the treatment of a preferred chemotherapeutic agent, Doxorubicin, which actually increased the lung metastasis. The demonstration that the treatment of VACNO reversed the lung metastasis induced by Doxorubicin would lead to the use of VACNO as an adjunctive therapy in breast cancer clinical trials where the mortality rate of breast cancer may be reduced through the inhibition of metastasis, especially in triple-negative patients.
3. The reduction in lung metastasis observed above is clearly related to the data showing that VACNO has direct effects on cancer cell proliferation and that this is associated with decreased cyclin D1 levels. Therefore, it is possible that cyclin D1 levels in tumors can serve as a marker for direct effects of VACNO on tumor growth. VACNO also has indirect effects on tumors by altering tumor blood flow. Increased blood flow is expected to reverse the hypoxia that is a hallmark in solid tumors. Hypoxia leads to activation of Hif1a and signaling through this pathway is known to control cancer metabolism, angiogenesis, proliferation, and cell survival. Furthermore, there is substantial evidence that hypoxia promotes cancer metastasis by influencing multiple steps in the process. Thus, Hif1a expression may serve as a marker of the indirect effects of VACNO on tumor blood flow and as an indicator of the efficacy of the drug against metastasis.
4. VACNO was shown to enhance the blood flow to the hypoxic core of solid flank TNBC model in a mouse suggested that the origin and driver of metastasis of tumor can be inhibited to improve the survival of cancer patients. Especially, the potential to improve survival of the cancer patient with multiple recurrence of metastasis due to ineffective standard cancer therapy like TNBC patients.
5. VACNO was shown to be efficacious in the inhibition of head neck cancer cells that is resistant to radiation and chemotherapy in vitro suggest that positioned VACNO in all body fluid of the cancer patients can reduce the dose requirements and side effect of cancer radiation therapy (results not shown).
6. VACNO administered through IP was shown to have the efficacy in the treatment of mouse model of brain cancer across the blood brain barrier similar to that in TNBC mouse model (results not shown). Therefore, give the high cost of treating human brain tumor without significant survival benefit, in this invention we propose to demonstrate VACNO can be used to prolong symptom free living in brain cancer patients with a continuous infusion pump for up to 5 years to deliver focal drug administration into the cerebral spinal fluid for the treatment of the proliferation, progression and metastasis of brain and spinal cancer.
7. VACNO was shown to reduce lung metastasis of a TNBC flank tumor caused by the standard breast cancer chemotherapy with Doxorubicin, which is also well known side effect. It is remarkable that IP VACNO is able to intercept the metastasis of escaped 4T1 cells caused by Doxorubicin to the lung. This preclinical efficacy data would suggest that conjunctive VACNO therapy with standard chemotherapy may improve the patient's survival.

The Claims of this invention are based on the specific preclinical results described here. More importantly this invention describes the translation of well established therapeutic mechanisms of VACNO from stroke and SCD to cancer therapy is based the extensive pre- clinical model studies including the inhibition of the extra vascular matrix breakdown in hemorrhagic transformation to the enhancement of blood flow without oxidative stress for focal ischemia in the brain. Furthermore, the anti-inflammatory, anti-ROS therapeutic activities and mechanisms of VACNO also apply in the inhibition of cancer carcinogenesis described in the current invention.

Metastases of tumors to multiple organs, rather than primary tumors, are responsible for 90% of the cancer deaths. To prevent these deaths, improved ways to treat metastatic disease are needed. Blood flow and other mechanical factors influence the delivery of cancer cells to specific organs, whereas molecular interactions between the cancer cells and the new organ influence the probability that the cells will grow there. Inhibition of the growth of metastases in secondary sites offers a promising approach for cancer cure.

Compared to acute stroke, the carcinogenesis leading to metastasis represents a complex series of chronic events which comprehensively covering many functional capabilities of a cell. These include tumor cell migration/proliferation and invasion into the tissues surrounding the primary tumor location, their subsequent intravasation into the blood or lymph vessels, extravasation back through vasculature to distal organs, and finally colonization/proliferation and angiogenesis of a new location to produce a second tumor body.

A growing body of scientific evidence has revealed that cancer surgery can increase the risk of metastasis. Even though this contradicts conventional medical thinking, the facts are undeniable. Therefore, the impact of metastasis from surgical removal of the primary tumor has to be addressed. The rationale of surgery is straightforward -- if you can get rid of the cancer by removing it from the body, then a cure can likely be achieved. Unfortunately, this approach does not take into account that following surgery, the cancer will frequently metastasize (spread to different organs). Quite often the metastatic recurrence is far more serious than the original tumor. In fact, for many cancers it is the metastatic recurrence, and not the primary tumor that ultimately proves to be fatal. This problem is most suitably addressed by the therapeutic mechanism of VACNO.

VACNO becomes strategically present in all extra cellular body fluids to track down isolated cancer cells that break away from the primary tumor. The following cellular events must be occurring in the presence of VACNO, first the cancer cell must breach the connective tissue immediately surrounding the tumor. Once this occurs, the cancer cell enters a blood or lymphatic vessel. To gain entry, the cancer cell must secrete enzymes that degrade the basement membrane of the blood vessel which are subject to inhibition by VACNO. This is vitally important for VACNO to inhibit the metastatic cancer cell as it uses the bloodstream and the intermediate steps for transport to other vital organs of the body (i.e., the liver, brain, or lungs) where it can form a new deadly tumor.

To complete its voyage, the cancer cell must adhere to the lining of the blood vessel where it degrades through and exits the basement membrane of the blood vessel. Its final task is to burrow through the surrounding connective tissue to arrive at its final destination, the organ. Now the cancer cell can multiply and form a growing colony, serving as the foundation for a new metastatic cancer. Fortunately, VACNO is capable of following the cancer cell metastasis each and every step along the way. Therefore, VACNO therapy is an ideal strategy to protect against the increased risk of metastasis.

The known activity of VACNO is its ability, through its nitroxide moieties, to dismutate superoxide and degrade hydrogen peroxide. In the vasculature, VACNO reduces superoxide, preventing it from reacting with nitric oxide and leading to enhanced blood flow.

Therefore, in this invention we present results below to show that VACNO would increase blood flow within tumors, leading to a reversal of tumor hypoxia. Hypoxia is a major factor in tumor metabolism, growth and metastasis (Rankin and Giaccia, 2016). Increased oxygenation of tumors is therefore expected to have significant consequences for tumorigenesis.

The 4T1 breast cancer model is a useful model for studying metastasis because it closely models human breast cancer and shows the same patterns of metastasis (Aslakson and Miller, 1992; Pulaski and Ostrand-Rosenberg, 1998; Yoneda et al., 2000). When injected into syngeneic mice primary 4T1 tumors grow rapidly and spontaneously metastasize to lung, bone, brain, and liver. We therefore used this model to examine the effects of VACNO on growth and metastasis of 4T1 mammary tumors. Cells were injected subcutaneously in the flank and tumors were allowed to develop for 4 days. Then the animals were treated with VACNO at 1 ml/Kg, 3 times per week. At end point, lungs were harvested from the animals and stained with India ink. Visible metastatic lung nodules were counted for each animal. VACNO treated animals showed a robust decrease in lung metastatic nodules. In another experiment tumor bearing animals were treated with Doxorubicin in the presence or absence of VACNO. Doxorubicin is commonly used to treat breast cancer patients. In the presence of Doxorubicin, VACNO treated mice also showed a significant reduction in lung metastases. In these experiments there was no significant difference in the growth of primary tumors. However, the finding of reduced metastasis if highly significant because it is metastatic disease that leads to more than 90% or all cancer related deaths.

Diagrams, Figures and Results are included to illustrate these paradigm shifts and showcase the therapeutic potency and efficacy of VACNO in inhibition of cancer cell proliferation, progression and metastasis in support the Claims of this patent application

In the following detailed description of Figures on VACNO for use in cancer, we have presented pre-clinical in vitro and in vivo efficacy data in TNBC. Similar results from head and neck squamous cell carcinoma (HNSCC) cancer (results not presented). The results of the TNBC studies which support the discovery of a novel therapeutic principle is disclosed in this invention. The utility of this invention of such NanoART to inhibit cancer metastasis may represent an improvement of "cure" for some of the human cancer patients with solid tumor. We describe below how the use of VACNO in therapy of preclinical model of TNBC may lead to major improvements in survival and quality of life for cancer patients.

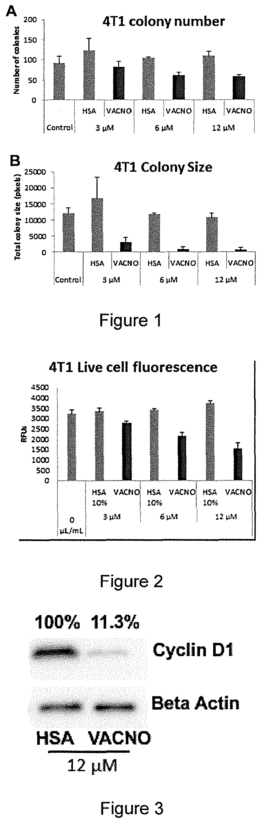

Reference is now made to Figure 1: In Vitro studies to examine direct effects of VACNO on cancer cells. These studies have been expanded to test in vitro VACNO concentrations across a broader dose range to define the optimize inhibition dose for inhibition of cancer cell proliferation. They have determined the inhibition effects of VACNO on at least 3 cell lines derived from different cancer types and from normal tissues. We have studies the mechanisms by which VACNO inhibits cancer cell proliferation by examining changes in intracellular levels of reactive oxygen species (ROS) in different cancerous cell lines and have examined the effects of VACNO on cell cycle regulatory proteins.

It is known that cancer cells characteristically have higher levels of ROS than normal tissues (Montero and Jassem, 2011; Wondrak, 2009). This is because they frequently are deficient in antioxidant enzymes but produce ROS at higher rates than normal cells. These persistently elevated ROS levels activate signaling mechanisms that are able to promote cell proliferation and tumor progression. For example, it is known that ROS is able to activate phosphoinositol-3 kinase (PI3K) and MAPK signaling pathways which can lead to activation of Rac1 and the stimulation of cell proliferation (Behrend et al., 2003; Huang et al., 2013).

VACNO possesses ROS catalytic breakdown activity and is able to eliminate superoxide and hydrogen peroxide, two key ROS produced by cells. Based on this information we have demonstrated that VACNO directly inhibited on cancer cell proliferation. In this study, a TNBC modeling 4T1 breast cancer cells were plated at low density and incubated for 1 week to allow them to form colonies. The cells were treated with a range of VACNO concentrations (Fig 1) and control cells were left untreated or treated with the same concentrations of human serum albumin (HSA). Compared to HSA treated cells, VACNO reduced the number of colonies formed, suggesting an effect on cell survival (Fig 1a). More strikingly, VACNO reduced colony size, suggesting that VACNO blocks cell proliferation (Fig 1b). These results have been confirmed by *direct* counting of cell number following VACNO treatment (not shown). The effects of VACNO on cell proliferation are observed at low micromolar concentrations of the drug.

Reference is now made to Figure 2. Since the known activity of VACNO is the ability to act as a superoxide dismutase mimetic and catalytic agent for ROS removal, we examined its effects on cancer cell ROS levels. Cells were treated with different concentrations of VACNO and then cellular ROS levels were estimated by staining with 2',7'-dichlorodihydro-fluorescein diacetate (OCF) followed by flow cytometry (Fig 2). The results demonstrate that VACNO causes a dose dependent decrease in cellular ROS levels.

Reference is now made to Figure 3. As an extension of our work showing that VACNO inhibits cancer cell proliferation we examined a variety of cell cycle regulatory proteins. We found that cyclin D1 levels are decreased in VACNO treated cancer cells (Fig 3). This finding is important because cyclin D1 is a documented oncogene product that is commonly upregulated in human cancers and is considered a potential target for therapy (Musgrove et al., 2011). In summary, VACNO appears to have direct effects on cancer cell proliferation. This is associated with reduced cellular ROS levels and the downregulation cyclin D1.

We have studied the VACNO inhibition of proliferation in head and neck squamous cell carcinoma (HNSCC) cell lines, mEER and MLM3 and have obtained similar results as shown in that of Fig. 1-3 (not shown). MLM3 is of special interest because it is resistant to cisplatin and radiation therapy (CRT) and forms highly metastatic tumors in vivo. The cell lines were analyzed against the effect of CRT treatment, VACNO was titrated across the same dose ranges and cell proliferation and survival determined by colony forming assays and direct cell counting as described above. Surprisingly, VACNO's anti-proliferation efficacy was by far more superior than CRT (results not shown). Like TNBC, HNSCC has a high incidence rate with nearly 600,000 cases annually worldwide. The incidence of HPV+ HNSCC is increasing and is associated with severe comorbidities. About ~10 % of these cases are resistant to standard therapies, develop metastases, and is an incurable disease. Our results would indicate that VACNO is such a potentially new and better treatments for this HNSCC patients.

These results are additional demonstrations that the inhibition of ROS with suppression of cyclin D1 expression is needed in addition to CRT treatment in order to fully inhibit cell proliferation in HNSCC patients.

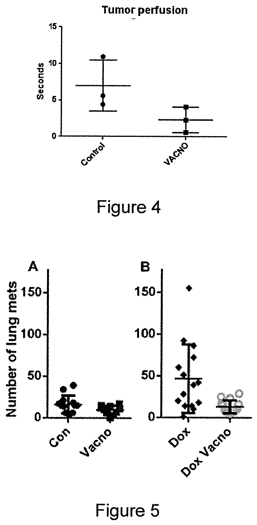

Reference is now made to Figure 4: Measurement of blood flow in 4T1 tumors in mice. Tumor bearing animals were injected with microbubbles and then with HAS or VACNO and time of tumor perfusion was measured by ultrasound. Lower time is indicative of faster blood flow through tumor.

VACNO promotes blood flow in tumors. The known activity of VACNO is its ability, through its nitroxide moieties, to dismutate superoxide and degrade hydrogen peroxide. In the vasculature, VACNO reduces superoxide, preventing it from reacting with nitric oxide and leading to enhanced blood flow. In Fig. 4 we have demonstrated that VACNO increases blood flow within tumors, leading to a reversal of tumor hypoxia. Hypoxia is a major factorin tumor metabolism, growth and metastasis (Rankin and Giaccia, 2016). Increased oxygenation of tumors is therefore expected to have significant consequences for tumorigenesis and metastasis.

Specifically, we have tested the effects of VACNO on blood flow, Balb/c mice were injected with 4T1 breast cancer cells and tumors were allowed to develop for 8 days. Then tumor diffusion time was analyzed by ultrasound. For this, microbubbles were injected through the tail vein, and a baseline reading of tumor perfusion was obtained. This was followed by VACNO or vehicle injection and a second reading of tumor perfusion time. Tumor perfusion was calculated by taking the 30 minute perfusion time minus the baseline time. A shorter time indicates greater perfusion of blood through the tumor. As shown in Fig 4, the perfusion time for tumors in VACNO treated animals was shorter than in vehicle treated animals. These results were confirmed by a second method using color Doppler ultrasound to image the tumor (not shown). Thus, as predicted VACNO significantly increases blood flow to tumors.

Reference is now made to Figure 5. Fig 5 shows the effect of VACNO on lung metastasis in 4T1 breast cancer model. A) Tumor bearing mice were treated with or without VACNO and after 3 weeks lungs were harvested for counting lung metastatic nodules. B) Same as in Fig 5A except that mice were also treated with doxorubicin. Metastatic nodules in the lung were significantly reduces with VACNO treatment (P <0.01).

Fig 5a shows that VACNO inhibits metastasis in a mouse model of breast cancer. The 4T1 breast cancer model is a useful model for studying metastasis because it closely models human breast cancer and shows the same patterns of metastasis (Aslakson and Miller, 1992; Pulaski and Ostrand-Rosenberg, 1998; Yoneda et al., 2000). When injected into syngeneic mice primary 4T1 tumors grow rapidly and spontaneously metastasize to lung, bone, brain, and liver. We have used this model to examine the effects of VACNO on growth and metastasis of 4T1 mammary tumors. Cells were injected subcutaneously in the flank and tumors were allowed to develop for 4 days. Then the animals were treated with VACNO at 1 ml/Kg 3 times per week. At end point, lungs were harvested from the animals and stained with India ink. Visible metastatic lung nodules were counted for each animal (Fig 5a). VACNO treated animals showed a robust decrease in lung metastatic nodules. In another experiment tumor bearing animals were treated with Doxorubicin in the presence or absence of VACNO. Doxorubicin is commonly used to treat breast cancer patients. In the presence of Doxorubicin, VACNO treated mice also showed a significant reduction in lung metastases (Fig 5b). In these experiments there was no significant difference in the growth of primary tumors. However, the finding of reduced metastasis if highly significant because it is metastatic disease that leads to more than 90% or all cancer related deaths.

### REFERENCES

1.Malorni L, Shetty PB, DeAngelis C, HilsenbeckS, Rimawi MF, Elledge R, Osborne CK, De Placido S, Arpino G. Clinical and biologic features of triple-negative breast cancers in a large cohort of patients with long-term follow-up. Breast Cancer Res Treat. 2012;136(3):795-804. doi: 10.1007/s10549-012-2315-y. PubMed PMID: 23124476; PMCID: PMC3513514.
2.Chikarmane SA, Tirumani SH, Howard SA, Jagannathan JP, DiPiro PJ. Metastatic patterns of breast cancer subtypes: what radiologists should know in the era of personalized cancer medicine. Clin Radial. 2015;70(1):1-10. doi: 10.1016/j.crad.2014.08.015. PubMed PMID: 25300558.
3.Maeda H. Toward a full understanding of the EPR effect in primary and metastatic tumors as well as issues related to its heterogeneity. Adv Drug Deliv Rev. 2015;91:3-6. doi: 10.1016/j.addr.2015.01.002. PubMed PMID: 25579058.
4.Montero AJ, Jassem J. Cellular redox pathways as a therapeutic target in the treatment of cancer. Drugs. 2011;71(11):1385-96. doi: 10.2165/11592590-000000000-00000. PubMed PMID: 21812504.
5.Mustacchi G, De Laurentiis M. The role of taxanes in triple-negative breast cancer: literature review. Drug Des Devel Ther. 2015;9:4303-18. doi: 10.2147/DDDT.S86105. PubMed PMID: 26273192; PMCID: PMC4532347.
6.Wondrak GT. Redox-directed cancer therapeutics: molecular mechanisms and opportunities. Antioxid Redox Signal. 2009;11(12):3013-69. doi: 10.1089/ARS.2009.2541. PubMed PMID: 19496700; PMCID: PMC2824519.
?.Behrend L, Henderson G, Zwacka RM. Reactive oxygen species in oncogenic transformation. Biochem Soc Trans. 2003;31(Pt6):1441-4. doi: 10.1042/. PubMed PMID: 14641084.
8.Huang JS, Cho CY, Hong CC, Yan MD, Hsieh MC, Lay JD, Lai GM, Cheng AL, Chuang SE. Oxidative stress enhances Axl-mediated cell migration through an Akt1/Rac1-dependent mechanism. Free Radic Bioi Med. 2013;65:1246-56. doi: 10.1016/j.freeradbiomed.2013.09.011. PubMed PMID: 24064382.
9.Musgrove EA, Caldon CE, Barraclough J, Stone A, Sutherland RL. Cyclin D as a therapeutic target in cancer. Nat Rev Cancer. 2011;11(8):558-72. doi: 10.1038/nrc3090. PubMed PMID: 21734724.
10.Rankin EB, Giaccia AJ. Hypoxic control of metastasis. Science. 2016;352(6282):175-80. doi: 10.1126/science.aaf4405. PubMed PMID: 27124451; PMCID: PMC4898055.
11. Aslakson CJ, Miller FR. Selective events in the metastatic process defined by analysis of the sequential dissemination of subpopulations of a mouse mammary tumor. Cancer Res. 1992;52(6):1399-405. PubMed PMID: 1540948.
12. Pulaski BA, Ostrand-Rosenberg S. Reduction of established spontaneous mammary carcinoma metastases following immunotherapy with major histocompatibility complex class II and B7.1 cell-based tumor vaccines. Cancer Res. 1998;58(7):1486-93. PubMed PMID: 9537252.
13.Yoneda T, Michigami T, Yi B, Williams PJ, Niewolna M, Hiraga T. Actions of bisphosphonate on bone metastasis in animal models of breast carcinoma. Cancer. 2000;88(12 Suppl):2979-88. PubMed PMID: 10898341.
14.Vyas D, Laput G, Vyas AK. Chemotherapy-enhanced inflammation may lead to the failure of therapy and metastasis. Onco Targets Ther. 2014; 7:1015-23. doi: 10.2147/0TI.S60114. PubMed PMID: 24959088; PMCID: PMC4061164.
15.Spano D, Heck C, De Antonellis P, Christofori G, Zollo M. Molecular networks that regulate cancer metastasis. Semin Cancer Bioi. 2012;22(3):234-49. doi: 10.1016/j.sem- cancer.2012.03.006. PubMed PMID: 22484561.
16.Haugrud AB, Zhuang Y, Coppock JD, Miskimins WK. Dichloroacetate enhances apoptotic cell death via oxidative damage and attenuates lactate production in metformin-treated breast cancer cells. Breast Cancer Res Treat. 2014. doi: 10.1007/s10549-014-3128-y. PubMed PMID: 25212175.
17.Zhuang Y, Miskimins WK. Cell cycle arrest in Metformin treated breast cancer cells involves activation of AMPK, downregulation of cyclin D1, and requires p27Kip1 or p21Cip1. J Mol Signal. 2008;3:18. Epub 2008/12/03. doi: 1750-2187-3-18 [pii] 10.1186/1750-2187-3-18. PubMed PMID: 19046439; PMCID: 2613390.
18.Zhuang Y, Miskimins WK. Metformin Induces Both Caspase-Dependent and Poly(ADP-ribose) Polymerase-Dependent Cell Death in Breast Cancer Cells. Mol Cancer Res. 2011;9:603-15. Epub 2011/03/23. doi: 1541-7786.MCR-10-0343 [pii] 10.1158/1541-7786.MCR-10-0343. PubMed PMID: 21422199.
19.Coppock JD, Vermeer PD, Vermeer DW, Lee KM, Miskimins WK, Spanos WC, Lee JH. mTOR inhibition as an adjuvant therapy in a metastatic model of HPV+ HNSCC. Oncotarget. 2016. doi: 10.18632/oncotarget.8286. PubMed PMID: 27015118.
20.Fuste NP, Fernandez-Hernandez R, Cemeli T, Mirantes C, Pedraza N, Rafel M, Torres-Rosell J, Colomina N, Ferrezuelo F, Dolcet X, Gari E. Cytoplasmic cyclin D1 regulates cell invasion and metastasis through the phosphorylation of paxillin. Nature communications. 2016; 7:11581. doi: 10.1038/ncomms11581. PubMed PMID: 27181366; PMCID: PMC4873647.
21.Lehn S, Tobin NP, Berglund P, Nilsson K, Sims AH, Jirstrom K, Harkonen P, Lamb R, Landberg G. Down-regulation of the oncogene cyclin 01 increases migratory capacity in breast cancer and is linked to unfavorable prognostic features. Am J Pathol. 2010;177(6):2886-97. doi: 10.2353/ajpath.2010.100303. PubMed PMID: 20971731; PMCID: PMC2993304.

## Claims

1. A nitroxylated macromolecule for use in the treatment of carcinogenesis and metastasis in the treatment of cancer in cancer patients comprising:
a) a macromolecule possessing anti-reactive oxygen species (ROS) activity
b) a macromolecule that enhances blood flow
c) a nitroxide that has a piperidine, pyrolidine or pyrolline ring
d) wherein the macromolecule is serum albumin.

2. The nitroxylated macromolecule for use according to claim 1, wherein, following administration, the nitroxylated macromolecule exerts anti-cancer effects in a body compartment into which it was not directly administered, including:
a) vascular system
b) cerebrospinal fluid
c) lymphatic system
d) skin
e) central nervous system
f) peripheral nervous system; and
g) any body organ.

3. The nitroxylated macromolecule for use according to claim 1, wherein said nitroxylated macromolecule is administered as a sole therapy, or in combination with another cancer drug or therapy.

4. The nitroxylated macromolecule for use according to claim 1, wherein on administration, the nitroxylated macromolecule localizes in all needed body fluid compartments to inhibit cancer carcinogenesis and metastasis at one or more locations in the body of the patient.

5. The nitroxylated macromolecule for use according to claim 1, wherein the nitroxylated macromolecule enhances blood flow or restores oxygen delivery to the hypoxic core of one or more tumors to inhibit carcinogenesis.

6. The nitroxylated macromolecule for use according to claim 1, wherein the nitroxylated macromolecule inhibits cancer cell proliferation and progression through the reduction of the cellular reactive oxygen species levels in cancer cells.

7. The nitroxylated macromolecule for use according to claim 1, wherein the nitroxylated macromolecule inhibits metastasis induced by conventional cancer therapies, thereby reducing mortality of cancer patients

8. The nitroxylated macromolecule for use according to claim 1, wherein the nitroxylated macromolecule inhibits target molecules responsible for cancer cell division thereby inhibiting cancer proliferation and progression.

9. The nitroxylated macromolecule for use according to claim 1, wherein, on administration, the nitroxylated macromolecule intercepts escaped cancer cells and inhibits proliferation, progression or metastasis of the cancer.

10. The nitroxylated macromolecule for use according to claim 1, wherein the polynitroxylated macromolecule is used in combination with a lipophilic cancer chemotherapeutic agent.

11. The nitroxylated macromolecule for use according to claim 1, wherein, on administration, the nitroxylated macromolecule distributes into all body fluid of the cancer patient to reduce the side effects of cancer radiation therapy and its induced metastasis as an undesirable side effect of radiation therapy.

12. The nitroxylated macromolecule for use according to claim 1, wherein the nitroxylated macromolecule is locally administered into the cerebral spinal fluid for treating one or more of the proliferation, progression and metastasis of brain or spinal cancer.

13. The nitroxylated macromolecule for use according to claim 1, wherein, on administration, the nitroxylated macromolecule is positioned in all body fluids of the cancer patients intercepting cancer cells escaped from a primary tumor to inhibit proliferation, progression and metastasis to secondary sites caused by standard chemotherapy or radiation therapy.

14. The nitroxylated macromolecule for use according to claim 1, wherein the number of nitroxides on the albumin molecule is between 40 - 60 nitroxides per molecule of albumin.

15. The nitroxylated macromolecule for use according to claim 1, wherein the nitroxylated macromolecule is complexed with paclitaxel and formed into nanoparticles of about 100-200 nm.

## Patentansprüche

1. Ein nitroxyliertes Makromolekül zur Verwendung bei der Behandlung von Karzinogenese und von Metastasierung bei der Behandlung von Krebs bei Krebspatienten, das Folgendes beinhaltet:
a) ein Makromolekül mit Aktivität gegen reaktive Sauerstoffspezies (ROS)
b) ein Makromolekül, das den Blutfluss verbessert
c) ein Nitroxid, das einen Piperidin-, Pyrrolidin- oder Pyrrolinring aufweist
d) wobei das Makromolekül Serumalbumin ist.

2. Nitroxyliertes Makromolekül zur Verwendung gemäß Anspruch 1, wobei das nitroxylierte Makromolekül nach Verabreichung Antikrebswirkungen in einem Körperkompartiment ausübt, in das es nicht direkt verabreicht wurde, umfassend:
a) Gefäßsystem
b) Rückenmarksflüssigkeit
c) Lymphsystem
d) Haut
e) zentrales Nervensystem
f) peripheres Nervensystem; und
g) ein beliebiges Körperorgan.

3. Nitroxyliertes Makromolekül zur Verwendung gemäß Anspruch 1, wobei das nitroxylierte Makromolekül als alleinige Therapie oder in Kombination mit einem anderen Krebsarzneimittel oder einer anderen Krebstherapie verabreicht wird.

4. Nitroxyliertes Makromolekül zur Verwendung gemäß Anspruch 1, wobei sich das nitroxylierte Makromolekül bei der Verabreichung in allen benötigten Körperflüssigkeitskompartimenten anordnet, um die Krebskarzinogenese und Metastasierung an einer oder mehreren Stellen in dem Körper des Patienten zu hemmen.

5. Nitroxyliertes Makromolekül zur Verwendung gemäß Anspruch 1, wobei das nitroxylierte Makromolekül den Blutfluss verbessert oder die Sauerstoffzufuhr zu dem hypoxischen Kern eines oder mehrerer Tumore wiederherstellt, um die Karzinogenese zu hemmen.

6. Nitroxyliertes Makromolekül zur Verwendung gemäß Anspruch 1, wobei das nitroxylierte Makromolekül die Proliferation und die Progression von Krebszellen durch die Reduktion der zellulären reaktiven Sauerstoffspeziesspiegel in Krebszellen hemmt.

7. Nitroxyliertes Makromolekül zur Verwendung gemäß Anspruch 1, wobei das nitroxylierte Makromolekül die durch herkömmliche Krebstherapien induzierte Metastasierung hemmt und dadurch die Sterblichkeit von Krebspatienten verringert.

8. Nitroxyliertes Makromolekül zur Verwendung gemäß Anspruch 1, wobei das nitroxylierte Makromolekül Zielmoleküle, die für die Krebszellteilung verantwortlich sind, hemmt, wodurch die Krebsproliferation und -progression gehemmt wird.

9. Nitroxyliertes Makromolekül zur Verwendung gemäß Anspruch 1, wobei das nitroxylierte Makromolekül bei Verabreichung ausgetretene Krebszellen abfängt und die Proliferation, Progression oder Metastasierung des Krebses hemmt.

10. Nitroxyliertes Makromolekül zur Verwendung gemäß Anspruch 1, wobei das polynitroxylierte Makromolekül in Kombination mit einem lipophilen chemotherapeutischen Krebsmittel verwendet wird.

11. Nitroxyliertes Makromolekül zur Verwendung gemäß Anspruch 1, wobei sich das nitroxylierte Makromolekül bei Verabreichung in allen Körperflüssigkeiten des Krebspatienten verteilt, um die Nebenwirkungen von Krebsbestrahlungstherapie und deren induzierte Metastasierung als eine unerwünschte Nebenwirkung der Bestrahlungstherapie zu verringern.

12. Nitroxyliertes Makromolekül zur Verwendung gemäß Anspruch 1, wobei das nitroxylierte Makromolekül lokal in die Gehirn-Rückenmarks-Flüssigkeit verabreicht wird, um eines oder mehrere von Proliferation, Progression und Metastasierung von Hirn- oder Rückenmarkskrebs zu behandeln.

13. Nitroxyliertes Makromolekül zur Verwendung gemäß Anspruch 1, wobei das nitroxylierte Makromolekül bei Verabreichung in allen Körperflüssigkeiten der Krebspatienten positioniert wird, wodurch Krebszellen abgefangen werden, die aus einem Primärtumor ausgetreten sind, um Proliferation, Progression und Metastasierung zu sekundären Stellen, die durch standardmäßige Chemotherapie oder Strahlentherapie verursacht werden, zu hemmen.

14. Nitroxyliertes Makromolekül zur Verwendung gemäß Anspruch 1, wobei die Anzahl der Nitroxide auf dem Albuminmolekül zwischen 40 und 60 Nitroxiden pro Albuminmolekül liegt.

15. Nitroxyliertes Makromolekül zur Verwendung gemäß Anspruch 1, wobei das nitroxylierte Makromolekül mit Paclitaxel komplexiert und zu Nanopartikeln von etwa 100-200 nm geformt ist.

## Revendications

1. Une macromolécule nitroxylée pour une utilisation dans le traitement de la carcinogenèse et des métastases dans le traitement d'un cancer chez des patients cancéreux comprenant :
a) une macromolécule possédant une activité anti-espèces réactives de l'oxygène (ERO)
b) une macromolécule qui renforce la circulation sanguine
c) un nitroxyde qui a un anneau pipéridine, pyrrolidine ou pyrroline
d) la macromolécule étant une albumine de sérum.

2. La macromolécule nitroxylée pour une utilisation selon la revendication 1, la macromolécule nitroxylée exerçant, suite à son administration, des effets anticancéreux dans un compartiment corporel dans lequel elle n'a pas été directement administrée, incluant :
a) le système vasculaire
b) le liquide céphalo-rachidien
c) le système lymphatique
d) la peau
e) le système nerveux central
f) le système nerveux périphérique ; et
g) tout organe du corps.

3. La macromolécule nitroxylée pour une utilisation selon la revendication 1, ladite macromolécule nitroxylée étant administrée en tant que thérapie unique, ou en combinaison avec un autre médicament ou une autre thérapie du cancer.

4. La macromolécule nitroxylée pour une utilisation selon la revendication 1, la macromolécule nitroxylée se localisant, après administration, dans tous compartiments de fluide corporel nécessaires afin d'inhiber la carcinogenèse et les métastases du cancer au niveau d'un ou de plusieurs endroits dans le corps du patient.

5. La macromolécule nitroxylée pour une utilisation selon la revendication 1, la macromolécule nitroxylée renforçant la circulation sanguine ou restaurant l'apport d'oxygène vers le coeur hypoxique d'une ou de plusieurs tumeurs afin d'inhiber la carcinogenèse.

6. La macromolécule nitroxylée pour une utilisation selon la revendication 1, la macromolécule nitroxylée inhibant la prolifération et l'évolution des cellules cancéreuses à travers la réduction des niveaux cellulaires d'espèces réactives de l'oxygène dans des cellules cancéreuses.

7. La macromolécule nitroxylée pour une utilisation selon la revendication 1, la macromolécule nitroxylée inhibant les métastases induites par des thérapies du cancer traditionnelles, réduisant de ce fait la mortalité de patients cancéreux.

8. La macromolécule nitroxylée pour une utilisation selon la revendication 1, la macromolécule nitroxylée inhibant des molécules cibles responsables de la division des cellules cancéreuses, inhibant de ce fait la prolifération et l'évolution du cancer.

9. La macromolécule nitroxylée pour une utilisation selon la revendication 1, la macromolécule nitroxylée interceptant, après administration, des cellules cancéreuses qui se sont échappées et inhibant la prolifération, l'évolution ou les métastases du cancer.

10. La macromolécule nitroxylée pour une utilisation selon la revendication 1, la macromolécule polynitroxylée étant utilisée en combinaison avec un agent chimiothérapeutique lipophile contre le cancer.

11. La macromolécule nitroxylée pour une utilisation selon la revendication 1, la macromolécule nitroxylée se répartissant, après administration, dans tout liquide organique du patient cancéreux afin de réduire les effets secondaires d'une radiothérapie du cancer et ses métastases induites en tant qu'effet secondaire indésirable de la radiothérapie.

12. La macromolécule nitroxylée pour une utilisation selon la revendication 1, la macromolécule nitroxylée étant administrée localement dans le liquide céphalo-rachidien pour traiter un ou plusieurs processus tels que la prolifération, l'évolution et les métastases d'un cancer du cerveau ou de la colonne vertébrale.

13. La macromolécule nitroxylée pour une utilisation selon la revendication 1, la macromolécule nitroxylée étant positionnée, après administration, dans tous liquides organiques des patients cancéreux, interceptant des cellules cancéreuses qui se sont échappées d'une tumeur primaire afin d'inhiber la prolifération, l'évolution et les métastases vers des sites secondaires causées par une chimiothérapie ou radiothérapie standard.

14. La macromolécule nitroxylée pour une utilisation selon la revendication 1, dans laquelle le nombre de nitroxydes sur la molécule d'albumine est de 40 à 60 nitroxydes par molécule d'albumine.

15. La macromolécule nitroxylée pour une utilisation selon la revendication 1, la macromolécule nitroxylée étant complexée avec du paclitaxel et formée en nanoparticules d'environ 100 à 200 nm.
